# EUROPEAN PATENT APPLICATION

(11) **EP 2 574 329 A1**
(43) Date of publication of application: **03.04.2013**
(21) Application number: 11183097.2
(22) Date of filing: 28.09.2011
(51) Int. Cl.: A61K 8/49, A61Q 15/00, A61K 31/34, A61P 17/00

(54) **Deodorant comprising dimethyl isosorbide**

(71) Applicant: ErlaCos GmbH, 91052 Erlangen (DE)
(72) Inventor: Windisch, Martin, 79189 Bad Krozingen (DE); Wieland, Heinrich, 79271 St. Peter (DE)
(74) Representative: Prüfer & Partner GbR European Patent Attorneys

(57) **Abstract**

Use of dimethyl isosorbide as an active deodorant agent in skin applications composition comprising dimethyl isosorbide for the treatment of bromhidrosis and a composition consisting of dimethyl isosorbide and water and optionally one or more of monohydric and polyhydric alcohols, metal salts, perfumes, colouring agents, oils, paraffins, triglycerides, surfactants and other conventional skin-care carrier substances without pharmacologic activity. Preferred compositions comprise dimethyl isosorbide, co-solvent e.g. water and/or ethanol and viscosity adjuster e.g. 1,2-pentanediol or polypropylene glycol.

## Description

The present invention relates to a new deodorant effective to reduce body odour.

Conventional deodorants for reducing body odour typically are compositions containing active ingredients besides carrier or vehicle components.

One type of active ingredients aim at controlling bacterial growth on the skin to be treated, since it is known that bacterial breakdown of perspiration substances cause bad odour, predominantly by releasing 3-methyl-2-hexeneoic by the bacterial's waste. Therefore, deodorants are often formulated with antimicrobials such as triclosan, or with metal-chelating agents which slow down bacterial growth.

Another type of active ingredients aim at exerting an antiperspirant activity. Typical antiperspirant active ingredients include aluminum substances and especially aluminum salts, such as aluminum chloride, aluminum chlorohydrate and aluminum-zirkonium compounds. The idea behind this deodorant concept resides in that aluminum-based complexes associate or react with components of the sweat to form plugs at the duct outlet of the sweat glands. The plugs shall prevent the gland from excreting liquid, thereby on the one hand preventing the provision of a substrate for bacterial breakdown, and on the other hand shall reduce perspiration and thus reduce moist climate in which bacteria tend to go.

Conventional deodorant compositions however suffer from significant drawbacks. In particular, the previously used active ingredients raise significant health concerns, such as the antimicrobials such as triclosan or metal-chelates, or the antipersipant aluminum substances hitherto used for deodorants. Aluminum substances furthermore readily produce unpleasant staining at the treated site of the skin.

The object of the present invention therefore was to provide an improved deodorant by providing a new concept to reduce body odour.

The inventor has surprisingly found that dimethyl isosorbide (which is known per se also in its abbreviated notations DMI or DMIS, or by its trade name "Arlasolve"; a basic structure being shown below) itself possesses remarkable deodorant activities and therefore can replace typical active ingredients conventionally used for deodorant compositions. This capacity of dimethyl isosorbide is unexpected, because albeit it has conventionally been applied as a (co-)solvent to be combined with active ingredients, for example for solubilising skin active ingredients or for promoting their penetration into or through the skin, and therefore has been conventionally used in formulations to increase the effectiveness of the active ingredient, it has not been identified as an active compound or its own for preventing or reducing bad body odour.

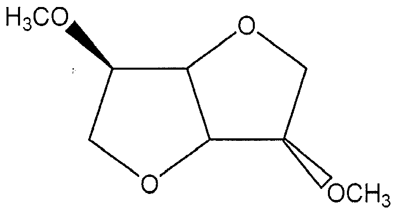

WO89/05143 discloses the use of a composition containing as the main active ingredient dimethyl isosorbide for the treatment of human skin. However unlike the specific situation of bad body odour, WO89/05143 contemplates only skin disorders unrelated to deodorant or antiperspirant activities, but rather includes a consideration of disorders like acne, impetigo, eczema, cellulitis, furuncles, carbuncles, folliculitis, tuberculosis of the skin, seborrhea, psoriasis, alopecia, mange, athlet's foot, herpes, warts, and corns.

As dimethyl isosorbide has been found according to the present invention to exhibit an immediate positive deodorant effect, it can be reasonably concluded that dimethyl isosorbide may associate with, or may mask, bad odour-causing substances such as 3-methyl-2-hexenoic acid. Moreover, it is likely that dimethyl isosorbide can act upon or cover sweat glands, either alone or upon association with biomolecules at the target site, thereby forming a deodorant/antiperspirant-combinatory effect.

Given its newly identified activities, it is to be appreciated that the use of dimethyl isosorbide as deodorant or in deodorant formulations does not suffer from drawbacks of conventionally used active ingredients or prior deodorant formulations. Known for long as a non-toxic and non-irritant substance, and in this sense conventionally used as a solvent or carrier in prior art skin or topic formulations, dimethyl isosorbide as deodorant can beneficially avoid conventional deodorant and antiperspirant active ingredients associated with strong health concerns. Further, the staining problem conventionally encountered with aluminum salts and compounds can be avoided also, if desired.

Therefore, the dimethyl isosorbide containing deodorant according to the present invention beneficially does not contain conventionally used deodorant active ingredients. Correspondingly, other antimicrobials such as triclosane and metal-chelating agents may be excluded, if desired according to preferred embodiments. More generally, the dimethyl isosorbide containing deodorant according to the present invention may, according to preferred embodiments, exclude other compounds of hitherto used deodorant active ingredients belonging to the classes of, e.g., other antiperspirants, odour absorbers, and enzyme inhibitors. Moreover, the deodorant according to the present invention may beneficially exclude germicide actives other than dimethyl isosorbide, thereby preferably excluding other anti-bacterial, anti-viral and anti-fungal active agents. The deodorant according to the present invention may also exclude the androsten-17(OR4)-4(R2)-3-one active ingredient of EP2060261A (R2 being hydrogen or OR5 with R5 being hydrogen or C1-C6 straight chain or branched alkyl; R4 being hydrogen or Cl-C6).

Hence, with the present invention, a deodorant is provided that is highly effective to reduce body odour, yet is well tolerated especially by the human skin.

If a combination with other deodorant active ingredients shall be used, a combination with metal salts is preferred, since the use of metal salts can beneficially co-act by achieving and maintaining a dry surrounding at the treated skin site, thereby inhibiting bacteria growth at the treated skin site. Suitable metal salts are salts of zinc, copper, tin and aluminum. Particular examples, belonging to the aluminum salt types, include but are not limited to aluminium chloride, aluminium chloride hexahydrate, and aluminium hydrochloride.

If a combination with other deodorant active ingredients shall be used, dimethyl isosorbide and the other deodorant active ingredient can be used in common or in separate skin administration forms, either at the same time or subsequently, respectively for a combined deodorant and/or antiperspirant purpose and use.

As the dimethyl isosorbide may itself exercise either or both of solvent and masking activities, it is even possible to omit, in preferred embodiments, potentially skin-irritating substances often used in conventional deodorant compositions, such as monohydric alcoholic solvents and perfumes. Moreover, due to the presence of dimethyl isosorbide while other active ingredients can be omitted, it is possible to avoid and therefore exclude stabilizing agents and preserving agents. Moreover, due to the simple constitution made feasible by the deodorant of the present invention, agents that have been conventionally often used in deodorants for improving texture or consistency, or anti-forming agents, can be beneficially avoided and excluded in the deodorant of the present invention, if desired.

On the other hand and depending on any one of the desire the producer or the end user, the form of application and the skin to be treated, it is possible to add any one selected from the following groups of substances alone or in combination: (i) monohydric alcohol solvents such as methanol, ethanol, propanol, isopropanol, butanol or longer alkyl-chain alcohols; (ii) perfumes and fragrancies; (iii) colouring agents; (iv) oils and paraffins; (v) surfactants such as Tween^{®} and Proloxamer^{®} (polyoxyethylene- polyoxypropylene-bloc copolymer), (vi) triglycerides and (vii) other conventional personal-care and skin-care carrier substances without pharmacologic activity. However, due to the activity of dimethyl isosorbide alone, it is not required to use either one of, and thereby to exclude anyone of, the substances of the respectively specific group (i), (ii), (iii), (iv), (v), (vi) or (vii).

In certain embodiments, the dimethyl isosorbide can be used as deodorant and/or antiperspirant alone, while in other and preferred embodiments it can be used in combination with a suitable solvent carrier. Solvent carriers may also enhance or prolong the deodorant and/or antiperspirant activity of dimethyl isosorbide. Suitable solvent carrier substances include, for example, co-solvents such as water, lower (C₁-C₆) alcohols, higher (C₆ or more) alcohols, and polyols. Polyhydric and especially monohydric alcohols may beneficially co-act with the deodorant and/or antiperspirant activity of dimethyl isosorbide. A preferred solvent carrier consists of water or aqueous solvent mixtures such as water with methanol, ethanol and/or isopropanol, preferably a water/ethanol mixture or a water/isopropanol mixture.

In an embodiment, dimethyl isosorbide is combined with at least one polyol, selected from 1,2-pentadiol, 1,5-pentadiol and polyglycols such as polyethylene glycol and polypropylene glycol. In this manner, it is possible to beneficially adjust the viscosity of a deodorant formulation.

The deodorant of the present invention can be provided in a suitable form as desired, typically as topical application forms. Suitable forms include, without being limited to, aqueous solutions, W/O and O/W emulsions, creams, ointments, sprays, as components of "roll-on" or stick-like deodorant devices, and the like, respectively arranged for topical use. The most preferred site of administration are the armpits and the feet of humans. When embodied in a deodorant formulation or composition, especially when combined with a solvent carrier, the amount of dimethyl isosorbide is suitably adjusted to be sufficient for reducing body odour and therefore to be effective as deodorant, for example at an amount of at least 0.5 wt.-%, preferably at least 5 wt.-% and more preferably at least 10 wt.-%. The maximum amount of dimethyl isosorbide in a formulation or composition depends on the desired product or field of application and suitably lies at 99 wt.-%, alternatively at maxima of 75 wt.-%, 50 wt.-%, 25 wt.-%, 10 wt.-%, or 5 wt.-%. The other substances of the deodorant formulation or composition of the present invention are chosen from the substances described above, optionally and beneficially excluding the undesirable substances mentioned above also.

In preferred embodiments, the deodorant of the present inventions consists only of dimethyl isosorbide and optionally water and a substance selected from monohydric and dihydric alcohols; and further optionally metal salts such as aluminum salt compounds. The optional alcohols being preferably selected from methanol, ethanol, 1,2-pentadiol, polyethylene glycol and polypropylene glycol, in particular ethanol and/or 1,2-pentadiol. Other active agents including other bactericides; other antiperspirants; other germicides; odor-absorbers; enzyme inhibitors; and metal-chelating agents are hence excluded in the present preferred embodiment. In this embodiment, a combination of advantages is feasible, namely reducing bad body odor, yet minimizing any potential toxicity, skin irritation or health concerns.

The deodorant according to the present invention, suitably formulated as a composition, can be used for cosmetic purposes only, for example for common personal-care or skin-care uses without a manifestation of a disease or pathologic condition. Alternatively, in severe situations of disease or pathologic conditions diagnosed as bromhidrosis, the deodorant and composition according to the present invention is suitable for use in a prophylactic and/or therapeutic treatment of bromhidrosis.

The following examples may illustrate the present invention without however being limited thereto.

### Example 1

A deodorant formulation is prepared as follows:

| | |
|---|---|
| dimethyl isosorbide | 7,5 wt.-% |
| ethanol | 50 wit.-% |
| polypropyleneglycol | 4 wit.-% |
| water | ad 100 wt.-% |

The formulation is used as a deodorant to be applied topically on the skin, for example by spraying with a conventional dispenser device.

### Example 2

A deodorant formulation is prepared from the following ingredients:

| | |
|---|---|
| dimethyl isosorbide | 10 wt.-% |
| 1,2-pentanediol | 2 wt.-% |
| aluminuniumchlorhydrate | 5 wt.-% |
| water | ad 100 wt.-% |

The formulation is used as deodorant and can be applied topically to the skin of armpits or the feet.

### Example 3

A deodorant is prepared by mixing the following ingredients:

| | |
|---|---|
| dimethyl isosorbide | 15 wt.-% |
| ethanol | 60 wt.-% |
| water | ad 100 wt.-% |

The formulation with good viscosity can be applied directly or via deodorant roll-on or stick devices to the target side such as armpits.

## Claims

1. Use of dimethyl isosorbide as an active deodorant agent in cosmetic skin applications.

2. Composition for use in a prophylactic and/or therapeutic treatment of bromhidrosis, the composition comprising dimethyl isosorbide.

3. The use according to claim 1 or the composition according to claim 2, wherein dimethyl isosorbide is used without other bactericides.

4. The use or composition according to claim 1, 2 or 3, wherein the dimethyl isosorbide is used without any one of the following active agents: other antiperspirants, other germicides; odor-absorbers; enzyme inhibitors; metal-chelating agents; and aluminum compounds.

5. The use or composition according to any one of the preceding claims, wherein a dimethyl isosorbide-containing composition further comprises any one selected from the group consisting of co-solvents and viscosity-adjusting agents.

6. The use or composition according to claim 5, wherein the co-solvent is selected from water and low-molecular (C1-C6) alcohol; and wherein the viscosity-adjusting agent is selected from diols, preferably pentane-diols, polyethylene glycol and polypropylene glycol.

7. The use or composition according to claim 5 or 6, the co-solvent is selected from the group consisting of water, ethanol and isopropyl alcohol.

8. The use or composition according to claim 5 or 6, wherein the viscosity-adjusting agent is 1,2-pentane-diol.

9. A composition consisting of dimethyl isosorbide and water, and optionally at least one substance selected from the following group of ingredients:
monohydric and polyhydric alcohols; metal salts; perfumes; colouring agents; oils and paraffins; triglycerides; surfactants; and other conventional skin-care carrier substances without pharmacologic activity.

10. The composition according to claim 9, wherein the optional further ingredient is limited to at least one alcoholic ingredient, and optionally a metal salt, while no further ingredient is added.

11. The composition according to claim 9 or 10, wherein at least one alcoholic ingredient is contained and is selected from methanol, ethanol, isopropyl alcohol, 1,2-pentadiol, polyethylene glycol and polypropylene glycol, in particular is ethanol, isopropyl alcohol and/or 1,2-pentadiol.
